# EUROPÄISCHE PATENTSCHRIFT

(11) **EP 1 831 147 B1**
(45) Veröffentlichungstag und Bekanntmachung des Hinweises auf die Patenterteilung: **02.12.2009**
(21) Anmeldenummer: 05819946.4
(22) Anmeldetag: 21.12.2005
(51) Int. Cl.: C07C 51/265, C07D 307/89

(54) **VERFAHREN ZUR HERSTELLUNG VON PHTHALSÄUREANHYDRID**
METHOD FOR PRODUCING PHTHALIC ANHYDRIDE
PROCEDE DE PRODUCTION D'ANHYDRIDE PHTALIQUE

(30) Priorität: 22.12.2004 DE 102004061770
(43) Veröffentlichungstag der Anmeldung: 12.09.2007
(73) Patentinhaber: BASF SE, 67056 Ludwigshafen (DE)
(72) Erfinder: HECHLER, Claus, 67063 Ludwigshafen (DE); OLBERT, Gerhard, 69221 Dossenheim (DE)
(74) Vertreter: Hörschler, Wolfram Johannes
(86) Internationale Anmeldenummer: PCT/EP2005/013774
(87) Internationale Veröffentlichungsnummer: WO 2006/069694

(56) Entgegenhaltungen:
- EP-A- 0 453 951
- DE-B- 1 274 569

## Beschreibung

Die Erfindung betrifft ein Verfahren zur Herstellung von Phthalsäureanhydrid durch katalytische Partialoxidation von o-Xylol und/oder Naphthalin mit einem Sauerstoff enthaltenden Gas in einer Anlage mit zwei oder mehreren gekühlten Reaktionszonen.

In der chemischen Verfahrenstechnik sind eine Vielzahl von Partialoxidationsreaktionen fluider, d.h. gasförmiger, flüssiger oder gasförmig/flüssiger Reaktionsgemische bekannt, die in Gegenwart von heterogenen partikelförmigen Katalysatoren durchgeführt werden. Derartige Umsetzungen sind in der Regel exotherm, häufig stark exotherm.

Phthalsäureanhydrid wird großtechnisch durch Partialoxidation von o-Xylol und/oder Naphthalin mit einem Sauerstoff enthaltenden Gas, häufig Luft, an heterogenen, insbesondere geträgerten Katalysatoren hergestellt. Die Reaktionsenthälpie beträgt für die Oxidation von o-Xylol 1110 kJ/mol und für Naphthalin 1792 kJ/mol gebildetes Phthalsäureanhydrid, d.h. die Reaktion ist stark exotherm. Für eine kostengünstige Produktion mit hohen Reaktordurchsätzen ist eine hohe Beladung der Luft mit Edukt erforderlich, häufig sind diese Gasmischungen dann zündfähig.

Zur Verringerung der Anteile an unerwünschten Zwischen-, Nebenprodukten sowie auch an Edukt im Prozessgas, die bei der Produktabtrennung aus dem Gasstrom und der weiteren Aufarbeitung Probleme bereiten, ist es meistens erforderlich Eduktumsätze nahe 100 Mol.-% einzustellen.

Wie auch bei anderen Partialoxidationen ist es bei der Herstellung von Phthalsäureanhydrid typisch, dass die Annäherung des Eduktumsatzes an 100% mit einer zunehmend höheren anteiligen Rate an Totaloxidation zu Kohlenmonoxid und Kohlendioxid verbunden ist. Auch können bei zu geringer Umsatzrate Nebenprodukte entstehen, im Falle der Synthese von Phthalsäureanhydrid insbesondere Phthalid, wodurch die Produktqualität beeinträchtigt wird. Aus den genannten Gründen muss nach dem Stand der Technik zur Erzeugung von spezifikationsgerechtem Produkt ein hoher Umsatz und somit ein spürbarer Ausbeuteverlust in Kauf genommen werden.

Aufgrund der Alterung des Katalysators mit zunehmender Betriebsdauer muss für einen gleichbleibenden Umsatz die Temperatur im Reaktor angehoben werden, wodurch nach dem Stand der Technik noch höhere Ausbeuteeinbußen hingenommen werden müssen.

In einem gewissen Maß kann die Ausbeute durch die Nutzung eines ungekühlten Nachreaktors verbessert werden, in welchem ein begrenzter Restumsatz bei erniedrigter Temperatur stattfindet, wodurch insbesondere auch die Einhaltung der Produktspezifikation ermöglicht wird. Am Ein- und Austritt des Nachreaktors enthält das Prozessgasgemisch noch sehr viel organische Substanz und auch freien Sauerstoff und ist in der Regel zündfähig. Zur Begrenzung des Temperaturanstiegs muss der Umsatz der Nachreaktion zur Vermeidung des Durchgehens der Reaktion strikt begrenzt werden.

Aus DE-A 101 44 857 ist eine Reaktoranordnung bekannt, bei der in einem einzigen Gehäuse ein als Rohrbündelreaktor ausgebildeter Hauptreaktor, eine Kühlstufe sowie ein Nachreaktor angeordnet sind, wobei der Nachreaktor ein ungekühlter Schachtreaktor ist. Durch diese Anordnung in einem Gehäuse ist das Gasvolumen zwischen Haupt- und Nachreaktionszone klein, mit dem Vorteil, dass das Zündrisiko dort stark reduziert wird. Es soll auf diese Weise eine vorteilhafte Ausbeute auch bei Beladungen von über 100 g o-Xylol pro Nm³ im Eduktgemisch realisierbar sein. Mit der vorgeschlagenen Betriebsweise, einer adiabaten Reaktionsführung im Nachreaktor, bleibt der dort mögliche Umsatz aus den oben schon genannten Gründen jedoch stark begrenzt, so dass auf diesem Weg weder eine signifikante Ausbeuteverbesserung und insbesondere keine Verlängerung der Katalysatorlebensdauer möglich ist. DE 1 274 569 offenbart eine Reaktoranordnung wobei Reaktionsrohr und Kühlelement einen gemeinsamen Mittelpunkt aufweisen.

Aus DE-A 40 13 051 ist ein Verfahren zur Herstellung von Phthalsäureanhydrid unter Verwendung eines Rohrbündelreaktors mit mindestens zwei in Strömungsrichtung aufeinanderfolgenden Reaktionszonen mit getrennter Salzbadkühlung bekannt, wobei die Salzbadtemperatur in der ersten Reaktionszone um 2 bis 20° höher gehalten wird als die Salzbadtemperatur in den verbleibenden Reaktionszonen. Durch diese Verfahrensführung wird eine verbesserte Ausbeute an Phthalsäureanhydrid erreicht, da die Temperaturführung besser an die reaktionskinetischen Abläufe angepasst ist. Trotzdem verbleiben hohe Ausbeuteverluste aufgrund des in beiden Kühlzonen hohen Temperaturniveaus, bei dem der erforderliche hohe Umsatz von ca. 99 % Mol.-% erreicht werden kann.

Es war demgegenüber Aufgabe der Erfindung, ein verbessertes Verfahren zur Herstellung von Phthalsäureanhydrid zur Verfügung zu stellen, wonach ein höherer Anteil des Restumsatzes in Verfahrensstufen, die der Synthese im Hauptreaktor nachgeschaltet sind, realisiert werden kann. Das Verfahren soll insbesondere auch flexibel an unterschiedliche Lästbereiche ohne Ausbeuteverluste angepasst werden können.

Die Lösung besteht in einem Verfahren zur Herstellung von Phthalsäureanhydrid durch katalytische Partialoxidation von o-Xylol und/oder Naphthalin mit einem Sauerstoff enthaltenden Gas in einer Anlage mit
- zwei oder mehreren mit einem Kühlmittel gekühlten Reaktionszonen,
- einer oder mehreren, zwischen den Reaktionszonen angeordneten Vorrichtungen zur Zwischenkühlung des Reaktionsgemisches zwischen den Reaktionszonen, wobei die Temperatur des Kühlmittels beim Eintritt in die zweite oder in die weiteren Reaktionszonen gegenüber der Temperatur des Kühlmittels beim Eintritt in die erste Reaktionszone abnimmt, das dadurch gekennzeichnet ist, dass die Temperatur des Kühlmittels beim Eintritt in die erste Reaktionszone um mehr als 20° höher ist als die Temperatur des Kühlmittels beim Eintritt in die zweite oder in die weiteren Reaktionszonen.

Es ist bekannt, dass die Umsatzrate in Nachreaktoren zur Herstellung von Phthalsäureanhydrid stark limitiert ist, da mit der Nachreaktion Wärme in den Reaktionsgasstrom eingetragen wird, diesen aufheizt und dann in der Folge die weitere Reaktion von bereits gebildetem Wertprodukt zu den Produkten der Totaloxidation, Kohlenmonoxid und Kohlendioxid, progressiv einsetzt, mit der Folge einer Minderausbeute sowie der Gefahr, dass der Nachreaktor durchgeht.

Es wurde überraschend gefunden, dass es, entgegen bisherigen Annahmen, möglich ist, einen nahezu vollständigen Umsatz von o-Xylol sowie des Nebenproduktes Phthalid zum Wertprodukt Phthalsäureanhydrid ohne merkliche, zusätzliche Ausbeuteverluste zu erreichen, sofern im Nachreaktor durch geeignete Wärmeabfuhr ein zu hoher Temperaturanstieg vermieden wird, selbst wenn Restumsätze im Bereich von 5 bis 10%, aber auch bis 15% oder auch bis 20% des molaren Gesamtumsatzes in den Nachreaktor verlagert werden, obgleich diese Nachreaktion auf einem Temperaturniveau deutlich unter dem zur Umsetzung von o-Xylol zu Phthalsäureanhydrid bei hohen Umsatzraten (> 70 %) als notwendig bekannten Temperaturniveau liegt.

Die Erfindung ist nicht eingeschränkt bezüglich der konkreten eingesetzten Katalysatoren: Es können alle bekannten Katalysatoren zur Herstellung von Phthalsäureanhydrid eingesetzt werden, insbesondere geträgerte Katalysatoren mit einer Aktivmasse enthaltend eines oder mehrere der Elemente Vanadium, Titan oder Phosphor, beispielsweise in Form ihrer Oxide.

Geeignete Katalysatoren sind beispielsweise in der Dissertation "In situ Charakterisierung von Vanadiumoxid/Titanoxid-Katalysatoren bei der partiellen Oxidation von o-Xylol zu Phthalsäureanhydrid" von Dipl.-Ing. M. Brust, Fakultät für Chemieingenieurwesen der Universität Karlsruhe, Tag des Kolloquiums: 20. Dezember 1999, und der dort referierten Literatur offenbart. Es ist auch möglich, Katalysatoren mit strukturierter Aktivität, d.h. mit unterschiedlicher Aktivität in einzelnen Reaktionszonen oder Bereichen hiervon, einzusetzen. Es können hierbei prinzipiell vollständig aus Aktivmasse bestehende so genannte Vollkatalysatoren, zum Beispiel in Kugel-, Zylinder-, Strang- oder Ringform aber auch mit Aktivmasse beschichtete Formkörper, zum Beispiel Kugeln oder Ringe eingesetzt werden. Die typischen Längenabmessungen bzw. Durchmesser dieser Formkörper sind im Bereich 1 mm bis 10 mm wobei häufiger 3 mm bis 8 mm und oft 5 mm bis 7 mm anzutreffen sind.

Besonders vorteilhaft kann der Katalysator in der ersten Reaktionszone beziehungsweise dem Hauptreaktor auf Ausbeute optimiert sein, da noch nennenswerte Anteile des Restumsatzes in nachgelagerten Reaktionszonen beziehungsweise im Nachreaktor erreicht werden können.

Als Edukt kann o-Xylol, Naphthalin oder ein Gemisch aus o-Xylol und Naphthalin eingesetzt werden.

Das für die Partialoxidation erforderliche Sauerstoff enthaltende Gas ist bevorzugt Luft.

Die zwei oder mehreren gekühlten Reaktionszonen können in einem einzigen Apparat untergebracht sein oder aber auch in zwei oder mehreren voneinander getrennten Apparaten. Besonders bevorzugt ist eine Ausführungsform mit zwei Reaktionszonen, einer ersten Reaktionszone in einem Hauptreaktor und einer zweiten Reaktionszone in einem Nachreaktor.

Die Reaktoren können zylindrisch sein, jedoch auch andere Geometrien aufweisen, beispielsweise parallelepipedisch, insbesondere quaderförmig, sein.

Die Kühlung der Reaktionszonen kann mit allen üblichen Wärmeträgern erfolgen. Besonders vorteilhaft können die Einrichtungen zur Aufnahme der Wärmeträger als Thermoblechplatten ausgebildet sein, durch die ein Kühlmittel strömt, bevorzugt Wasser, das hierbei verdampft (sogenannte Siedekühlung).

Zwischen den Reaktionszonen sind Zwischenkühler für das Reaktionsgasgemisch angeordnet.

Das Verfahren kann besonders vorteilhaft in einem Apparat mit Thermoblechplatten durchgeführt werden, wobei Hauptreaktor, Zwischenkühler und Nachreaktor im selben Apparat angeordnet sind. Hierbei sind im Hauptreaktor sowie im Nachreaktor in den Zwischenräumen zwischen den Thermoblechplatten Schüttungen des Feststoffkatalysators eingebracht, über die das Reaktionsgasgemisch geleitet wird.

Im Zwischenkühler kann vorzugsweise eine Inertschüttung eingebracht sein. Es ist auch möglich, den Zwischenkühler und den Nachreaktor nur dadurch zu differenzieren, dass der Bereich, der als Zwischenkühler fungiert freigelassen wird oder dass darin eine Inertschüttung eingebracht ist und dass in dem Bereich, der als Nachreaktor fungiert, eine Schüttung als Feststoffkatalysator eingebracht ist. Vorzugsweise werden der Zwischenkühler und der Nachreaktor an einen einzigen Kühlkreis angeschlossen, der vom Kühlkreis für den Hauptreaktor getrennt ist.

Die Begriffe Wärmetauscherplatten, Wärmeübertragerplatten, Thermobleche, Thermoplatten oder Thermoblechplatten werden weitgehend synonym verwendet.

Wärmeübertragerplatten werden überwiegend als flächenförmige Gebilde definiert, die einen mit Zu- und Abführleitungen versehenen Innenraum mit geringer Dicke im Verhältnis zur Fläche aufweisen. Sie werden in der Regel aus Blechen, häufig aus Stahlblechen, hergestellt. Je nach Anwendungsfall, insbesondere den Eigenschaften des Reaktionsmediums sowie des Wärmeträgers, können jedoch spezielle, insbesondere korrosionsfeste, aber auch beschichtete Werkstoffe zum Einsatz kommen. Die Zu- bzw. Abführeinrichtungen für die Wärmeträger sind in der Regel an einander entgegengesetzten Enden der Wärmetauschplatten angeordnet. Als Wärmeträger kommen häufig Wasser, auch Diphyl^{®} (Gemisch aus 70 bis 75 Gew.-% Diphenylether und 25 bis 30 Gew.-% Diphenyl) zum Einsatz, welche auch teilweise in einem Siedevorgang verdampfen; es ist auch der Einsatz anderer organischer Wärmeträger mit niedrigem Dampfdruck ionischer Flüssigkeiten möglich.

Die Verwendung ionischer Flüssigkeiten als Wärmeträger ist in DE-A 103 16 418 beschrieben. Bevorzugt sind ionische Flüssigkeiten, die ein Sulfat-, Phosphat-, Borat- oder Silikatanion enthalten. Besonders geeignet sind auch ionische Flüssigkeiten, die ein einwertiges Metall-Kation, insbesondere ein Alkalimetall-Kation, sowie ein weiteres Kation, insbesondere ein Imidazolium-Kation, enthalten. Vorteilhaft sind auch ionische Flüssigkeiten, die als Kation ein Imidazolium-, Pyridinium- oder Phosphonium-Kation enthalten.

Der Begriff Thermobleche oder Thermoblechplatten wird insbesondere für Wärmeübertragerplatten verwendet, deren einzelne, meistens zwei, Bleche durch Punkt- und/oder Rollschweißungen miteinander verbunden und häufig unter Verwendung hydraulischen Drucks plastisch unter Kissenbildung ausgeformt sind.

Bevorzugt kann zur Durchführung des erfindungsgemäßen Verfahrens ein Reaktor eingesetzt werden, wie er in DE 103 33 866 beschrieben ist, deren Offenbarungsgehalt hiermit vollumfänglich in die vorliegende Patentanmeldung einbezogen wird, d.h. ein Reaktor mit
- einem oder mehreren quaderförmigen Thermoblechplattenmodulen, die jeweils aus zwei oder mehreren rechteckigen, parallel zueinander unter Freilassung jeweils eines Spaltes angeordneten Thermoblechplatten gebildet sind, der mit dem heterogenen partikelförmigen Katalysator befüllbar ist und der vom fluiden Reaktionsgemisch durchströmt wird, wobei die Reaktionswärme von einem Wärmeträger aufgenommen wird, der die Thermoblechplatten durchströmt und dabei zumindest teilweise verdampft, sowie mit
- einer die Thermoblechplattenmodule druckentlastenden, dieselben vollständig umgebenden überwiegend zylinderförmigen Hülle, umfassend einen Zylindermantel und denselben an beiden Enden abschließenden Hauben und deren Längsachse parallel zur Ebene der Thermoblechplatten ausgerichtet ist, sowie mit
- einem oder mehreren Abdichtelementen, die dergestalt angeordnet sind, dass das fluide Reaktionsgemisch außer durch die von den Hauben begrenzten Reaktorinnenräume nur durch die Spalte strömt.

Es handelt sich somit um Thermoblechplattenmodule mit Thermoblechplatten, durch die ein Wärmeträger strömt, der die Reaktionswärme aufnimmt und hierbei zumindest teilweise verdampft, die quaderförmig ausgebildet sind und druckentlastend in einer dieselben vollständig umgebenden überwiegend zylinderförmigen Hülle eingebracht sind.

Die Blechplattenmodule sind aus jeweils zwei oder mehreren rechteckigen, parallel zueinander unter Freilassung jeweils eines Spaltes angeordneten Thermoblechplatten gebildet.

Die Thermoblechplatten sind aus korrosionsresistenten Werkstoffen, vorzugsweise aus Edelstahl, beispielsweise mit der Werkstoffnummer 1.4541 bzw. 1.4404, 1.4571 bzw. 14406, 1.4539 aber auch 1.4547 oder aus anderen legierten Stählen, gefertigt.

Die Materialstärke der hierfür eingesetzten Bleche kann zwischen 1 und 4 mm, 1,5 und 3 mm, aber auch zwischen 2 und 2,5 mm, oder zu 2,5 mm oder zu 3 mm gewählt werden.

In der Regel werden zwei rechteckige Bleche an ihren Längs- und Stirnseiten zu einer Thermoblechplatte verbunden, wobei eine Rollnaht oder seitliches Zuschweißen oder eine Kombination von beidem möglich ist, so dass der Raum, in dem sich später der Wärmeträger befindet, allseitig dicht ist. Vorteilhaft wird der Rand der Thermoblechplatten an oder schon in der seitlichen Rollnaht der Längskante abgetrennt, damit der schlecht oder nicht gekühlte Randbereich, in dem meist auch Katalysator eingebracht ist, eine möglichst geringe geometrische Ausdehnung hat.

Über die Rechteckfläche verteilt werden die Bleche miteinander durch Punktschweißung verbunden. Auch eine zumindest teilweise Verbindung durch gerade oder auch gebogene und auch kreisförmige Rollnähte ist möglich. Auch die Unterteilung des vom Wärmeträger durchströmten Volumens in mehrere getrennte Bereiche durch zusätzliche Rollnähte ist möglich.

Eine Möglichkeit der Anordnung der Schweißpunkte auf den Thermoblechplatten ist in Reihen mit äquidistanten Punktabständen von 30 bis 80 mm oder auch 35 bis 70 mm, wobei auch Abstände von 40 bis 60 mm möglich sind, wobei eine weitere Ausführungsform Abstände von 45 bis 50 mm und auch 46 bis 48 mm ist. Typischerweise variieren die Punktabstände fertigungsbedingt bis zu ± 1 mm und die Schweißpunkte unmittelbar benachbarter Reihen in Längsrichtung der Platten gesehen, jeweils um einen halben Schweißpunktabstand versetzt angeordnet. Die Reihen der Punktschweißungen in Längsrichtung der Platten können äquidistant mit Abständen von 5 bis 50 mm, aber auch von 8 bis 25 mm, wobei auch Abstände von 10 bis 20 mm und auch 12 bis 14 mm, eingesetzt werden. Weiterhin sind auch dem Anwendungsfall angepasste Paarungen der genannten Schweißpunktabstände und Reihenabstände möglich. Die Reihenabstände können in einem definierten geometrischen Zusammenhang zum Punktabstand, typisch ¼ der Punktabstände oder etwas geringer sein, so dass sich eine definiert gleichmäßige Aufweitung der Thermobleche bei der Herstellung ergibt. Vorgegebenen Schweißpunkt- und Reihenabständen ist eine entsprechend Anzahl von Schweißpunkten je m² Plattenoberfläche zugeordnet.

Die Breite der Thermoblechplatten ist im Wesentlichen fertigungstechnisch begrenzt und kann zwischen 100 und 2500 mm, oder auch zwischen 500 und 1500 mm, liegen. Die Länge der Thermoblechplatten ist abhängig von der Reaktion, insbesondere vom Temperaturprofil der Reaktion, und kann zwischen 500 und 7000 mm, oder auch zwischen 3000 und 4000 mm liegen.

Jeweils zwei oder mehrere Thermoblechplatten sind parallel und beabstandet zueinander, unter Bildung eine Thermoblechplattenmodules, angeordnet. Dadurch entstehen zwischen unmittelbar benachbarten Blechplatten schachtartige Spalte, die an den engsten Stellen des Plattenabstandes beispielsweise eine Breite zwischen 8 und 150 mm, aber auch 10 bis 100 mm aufweisen. Eine mögliche Ausführung sind auch Breiten von 12 bis 50 mm oder aber 14 bis 25 mm, wobei auch 16 bis 20 mm gewählt werden können. Es wurde auch schon ein Spaltabstand von 17 mm erprobt.

Zwischen den einzelnen Thermoblechplatten eines Thermoblechplattenmodules können, z.B. bei großflächigen Platten, zusätzlich Distanzhalter eingebaut werden, um Verformungen vorzubeugen, welche Plattenabstand oder -position verändern können. Zum Einbau dieser Distanzhalter können Teilbereiche der Bleche durch zum Beispiel kreisförmige Rollnähte vom Durchflussbereich des Wärmeträgers abgetrennt werden, um dort beispielsweise Löcher für Befestigungsschrauben der Distanzhalter in die Platten einbringen zu können.

Die Spalte können gleichen Abstand besitzen, bei Erfordernis können die Spalte aber auch unterschiedlich breit sein, wenn die Reaktion dies zulässt oder die gewünschte Reaktion es erfordert, oder apparative oder kühltechnische Vorteile erzielt werden können.

Die mit Katalysatorpartikeln gefüllten Spalte eines Thermoblechplattenmodules können gegeneinander gedichtet, zum Beispiel dichtgeschweißt sein oder auch prozessseitig zueinander Verbindung besitzen, so dass zwischen den mit Prozessgas durchströmten Spalten ein Druckausgleich stattfinden kann.

Zur Einstellung des gewünschten Spaltabstandes beim Zusammenfügen der einzelnen Thermoblechplatten zu einem Modul werden die Platten in ihrer Position und im Abstand fixiert. Eine Möglichkeit ist der Einsatz zweier seitlicher Begrenzungsplatten für jedes Modul, an oder in denen die einzelnen Thermoblechplatten durch formschlüssige Fixierung z.B. durch Einführung der seitlichen Plattenkanten in vorher eingebrachte Nuten, Schlitze oder auch durch stoffschlüssige Fixierung wie Schweißen auf einer ebenen Platte erfolgt, wobei über die gesamte Länge der Platte dichtgeschweißt oder zumindest teilweise auch nur geheftet werden kann, um einen Druckausgleich der Spalte zu ermöglichen. Eine alternative Ausführungsform ist die Verwendung von rohrförmigen, gewinkelten oder gekrümmten Blechteilen, die jeweils zwischen zwei Platten geheftet und nach außen hin dichtgeschweißt werden.

Die Schweißpunkte unmittelbar benachbarter Thermoblechplatten können sich gegenüberliegen oder versetzt zueinander sein.

In der Regel wird es aus fertigungstechnischen Gründen bevorzugt sein, bei der Anordnung mit zwei oder mehreren quaderförmigen Thermoblechplattenmodulen, dieselben mit jeweils gleichen Abmessungen auszubilden. Bei Anordnungen von 10 oder 14 Thermoblechplattenmodulen kann es für die Kompaktheit des Gesamtapparates vorteilhaft sein, zwei Modultypen mit unterschiedlicher Kantenlänge bzw. unterschiedlichem Kantenlängenverhältnis zu wählen.

Bevorzugt sind Anordnungen von 4, 7, 10 oder 14 Thermoblechplattenmodulen mit jeweils gleichen Abmessungen. Die in Strömungsrichtung sichtbare Projektionsfläche eines Moduls kann quadratisch sein, aber auch rechteckig ist einem Seitenverhältnis von 1,1 aber auch 1,2. Vorteilhaft sind Kombinationen von 7, 10 oder 14 Modulen mit rechteckigen Modulprojektionen, so dass der Durchmesser der äußeren zylindrischen Hülle minimiert wird. Besonders vorteilhafte geometrische Anordnungen sind erzielbar, wenn, wie oben aufgeführt, eine Anzahl von 4, 7 oder 14 Thermoblechplattenmodulen gewählt wird.

Vorteilhaft sollen hierbei die Thermoblechplattenmodule einzeln auswechselbar sein, beispielsweise bei Leckagen, Verformungen der Thermobleche oder bei Problemen, die den Katalysator betreffen.

Vorteilhaft sind die Thermoblechplattenmodule in jeweils einem druckstabilen, rechteckigen Stabilisierungskasten angeordnet.

Jedes Thermoblechplattenmodul wird vorteilhaft durch eine geeignete Führung, beispielsweise durch die rechteckigen Stabilisierungskästen, mit seitlich durchgehender Wandung oder beispielsweise durch eine Winkelkonstruktion in Position gehalten.

In einer Ausführungsform sind die rechteckigen Stabilisierungskästen benachbarter Thermoblechplattenmodule gegeneinander abgedichtet. Dadurch wird eine Bypass-Strömung des Reaktionsgemisches zwischen den einzelnen Thermoblechplattenmodulen verhindert.

Durch den Einbau von quaderförmigen Thermoblechplattenmodulen in eine überwiegend zylindrische drucktragende Hülle verbleiben am Rand zur zylindrischen Mantelwand der Hülle relativ große freie Zwischenräume, in denen eine Ablagerung, Nebenreaktionen oder eine Zersetzung des Werkproduktes stattfinden können. Eine Reinigung, Dekontamination von Produkt zum Beispiel bei der Notwendigkeit von Montagetätigkeiten ist dort nur stark erschwert möglich. Es ist daher vorteilhaft, diesen Zwischenraum vom Reaktionsraum, das heißt von den Spalten zwischen jeweils unmittelbar benachbarten Thermoblechplatten, zu separieren.

Hierzu verschließt man den Zwischenraum zwischen den Thermoblechplattenmodulen und der überwiegend zylinderförmigen Hülle am unteren Ende der Thermoblechplattenmodule mit einem Halteboden. Um eine Bypass-Strömung des Reaktionsgemisches zu verhindern, soll der Trag- oder Halteboden den Zwischenraum gasdicht verschließen.

Vorteilhaft kann der Zwischenraum zwischen den Thermoblechplattenmodulen und der überwiegend zylinderförmigen Hülle auch am oberen Ende der Thermoblechplattenmodule durch eine Blechabdeckung verschlossen werden. Hierfür ist jedoch ein gasdichter Verschluss nicht notwendig, es ist in einer Ausführungsform möglich, die Blechabdeckung mit Öffnungen auszubilden.

Die Blechabdeckung am oberen Ende des Zwischenraumes zwischen den Thermoblechplattenmodulen und der überwiegend zylinderförmigen Hülle kann vorteilhaft auch ähnlich einem Ventilboden ausgebildet sein.

Die Abströmung des zur Druckbeaufschlagung verwendeten Gases kann weiterhin mittels eines Überströmorgans, als Blende, Ventil oder kraftbelastetes (zum Beispiel mit Feder oder Gasdruck), selbstregelndes Organ ausgeführt, auch in Kombination mit einer Rückschlagsicherung, hergestellt werden. Diese Überströmorgane können auch außerhalb der zylindrischen Außenhülle angeordnet sein.

Die obere Blechabdeckung kann auf Verstrebungen sitzen, welche die rechteckigen Stabilisierungskästen, in denen Thermoblechplattenmodule eingebracht sind, zusätzlich stabilisieren.

Der Zwischenraum zwischen den Thermoblechplattenmodulen und der vorwiegend zylinderförmigen Hülle kann vorteilhaft mit Inertstoffen ausgefüllt werden, um das freie Gasvolumen zu verringern und um Gaskonvektion zu verhindern, welche beispielsweise zu unkontrolliertem Wärmeabfluss führen kann.

In der zylinderförmigen Hülle sind vorteilhaft Stutzen für die Zu- und Abführung des Inertstoff-Schüttgutes vorgesehen, die in geeigneter Größe ausgeführt und Neigung angebracht sind, so dass eine staufreie Befüllung und Entleerung schwerkraftgetrieben möglich ist. Mögliche Ausführungsformen der Stutzen sind Nennweiten von 80, 100, 150 oder 200 mm.

Als Inertstoff-Schüttgut kann grundsätzlich jedes chemisch inerte und mechanisch und thermisch ausreichend stabile Material eingesetzt werden, beispielsweise expandierter Perlite und/oder expandierter Vermiculit.

Es ist möglich, den Zwischenraum zwischen den Thermoblechplattenmodulen und der überwiegend zylinderförmigen Hülle, der mit Inertmaterial ausgefüllt sein kann, mit einem Gasdruck zu beaufschlagen.

Die Druckbeaufschlagung kann im Wesentlichen statisch sein und vorteilhaft durch die druckgeregelte Zu- und Abfuhr von Stickstoff bewirkt werden. Als Regelsignal kann beispielsweise der Druckunterschied zwischen dem Druck im Zwischenraum zwischen den Thermoblechplattenmodulen und der überwiegend zylinderförmigen Hülle und der Druck am unteren Ende der Katalysatorschüttung in den Spalten der Thermoblechplattenmodule oder am oberen Ende desselben gewählt werden. Vorteilhaft kann das Differenzdrucksignal um einen Offsetwert korrigiert sein, bevorzugt kann ein Mittelwert, insbesondere der arithmetische Mittelwert des Druckes über die Höhe der Katalysatorschüttung als Regelsignal gewählt werden.

Zur Druckbeaufschlagung können in der überwiegend zylinderförmigen Hülle entsprechende Stutzen und/oder eine interne Ringleitung mit kleinen Bohrungen vorgesehen sein, die vorzugsweise nach unten gerichtet sind.

Alternativ ist es auch möglich, die Druckbeaufschlagung unter kontinuierlicher Durchströmung des Zwischenraumes mit einem prozessinerten oder prozessintrinsischen Gas, insbesondere mit Stickstoff oder mit Kreisgas zu bewirken.

Das zur Druckbeaufschlagung verwendete Gas wird vorteilhaft mit dem fluiden Reaktionsgemisch an dessen Austritt aus den Thermoblechplattenmodulen vereinigt, in der Regel noch innerhalb der überwiegend zylinderförmigen Hülle des Reaktors. Die Ausströmstellen des zur Druckbeaufschlagung verwendeten Gases sind vorteilhaft in Strömungstotzonen des fluiden Reaktionsgemisches gelegt, um dieselben zu spülen.

Der Volumenstrom des zur Druckbeaufschlagung verwendeten Gases wird in der Regel signifikant kleiner sein als der Volumenstrom des fluiden Reaktionsgemisches und wird vorteilhaft so gewählt werden, dass er für die Reaktion prozesstechnisch unschädlich ist.

Die Thermoblechplattenmodule sollen vorteilhaft jeweils einzeln austauschbar sein, um, wie vorstehend bereits ausgeführt, bei auftretenden Problemen, beispielsweise Leckagen, Verformungen der Thermobleche oder Problemen mit dem Katalysator, gezielt Abhilfe schaffen zu können. Hierfür ist es vorteilhaft, die Thermoblechplattenmodule mit etwas Spiel gegenüber der Wandung der rechteckigen Stabilisierungskästen auszubilden.

Da die Thermoblechplattenmodule somit bei dieser vorteilhaften Ausführung nichtdichtend in den rechteckigen Stabilisierungskästen sitzen, kann es zu Bypass-Strömungen des Reaktionsmediums kommen. Um dies zu vermeiden, werden die Undichtigkeiten zwischen den Thermoblechplattenmodulen und den rechteckigen Stabilisierungskästen in geeigneter Weise abgedichtet, beispielsweise mit auf der Außenseite der Thermoblechplattenmodule angebrachten Metallblechstreifen, die sich beim Einschieben in den rechteckigen Stabilisierungskasten an die Wand desselben drücken. Alternativ sind gasdichte Blechabdeckungen und -verbindungen, beispielsweise in Form von Schweißlippen-Dichtungen möglich.

Nach dem Einschieben der Thermoblechplattenmodule in die rechteckigen Stabilisierungskästen können dieselben gegen den Halteboden abgedichtet werden, der den Zwischenraum zwischen den Thermoblechplattenmodulen und der überwiegend zylinderförmigen Hülle am unteren Ende der Thermoblechplattenmodule verschließt. Hierfür ist grundsätzlich jede bekannte Dichtungsmöglichkeit einsetzbar. Es kann sich um herkömmliche Dichtungen handeln, die beispielsweise zusätzlich verschraubt sind.

Es ist auch möglich, die Abdichtung durch Schweißlippen zu bewirken, beispielsweise durch eine Variante, bei der eine Schweißlippe am Halteboden befestigt ist und eine zweite Schweißlippe am Außenrand des Thermoblechplattenmodules oder des rechteckigen Stabilisierungskastens. Beide Schweißlippen sind so gestaltet, dass sie geometrisch zusammenpassen und zusammengeschweißt werden können. Zum Austausch des Thermoblechplattenmodules wird die Schweißnaht aufgetrennt und bei Bedarf erneuert.

Die Thermoblechplattenmodule können durch eine Einrichtung von oben mit den rechteckigen Stabilisierungskästen verspannt werden. Durch genügend Spanndruck von oben wird eine ausreichende Flächenpressung auf der Dichtung erreicht und die Thermoblechplattenmodule werden vorteilhaft fixiert.

Die rechteckigen Stabilisierungskästen müssen nicht zwingend gegeneinander abgedichtet sein, solange eine unzulässige Bypassströmung an den Spalten vorbei verhindert wird. Es ist auch möglich, die rechteckigen Stabilisierungskästen untereinander mit kleinen Bohrungen zu verbinden, durch die Inertgas aus dem Zwischenraum zwischen Thermoblechplattenmodulen und der überwiegend zylinderförmigen Hülle einströmen kann, wodurch im Raum zwischen dem Thermoblechplattenmodul und dem rechteckigen Stabilisierungskasten Reaktionen verhindert werden.

Die Thermoblechplattenmodule können zusätzlich auch an der Außenseite Führungs- und Ausrichtungselemente aufweisen. Es ist beispielsweise möglich, an den Ecken derselben Eckwinkel jeglicher Form und an ihrer Seite konische Blechstreifen vorzusehen. Weiterhin ist es von Vorteil an den Modulen Anschlagvorrichtungen oder Anschlaghilfsmittel wie Ösen, Laschen oder Gewindebohrungen anzubringen, um ein einfaches Einsetzen mittels eines Hebezeugs oder z. B. eines Krans zu ermöglichen. Zum Einkranen der Thermoblechplattenmodule können diese auch an Zugankern gehalten werden, welche vertikal durch die zunächst leeren Spalte bis zur Unterkante der Platten reichen und dort mit einem Querträger zur Lastaufnahme verbunden sind.

In einer besonderen Ausführungsform kann die äußerste Thermoblechplatte eines Thermoblechplattenmodules an der äußeren Seite derselben aus einem gegenüber den übrigen zur Herstellung der Thermoblechplatten eingesetzten Blechen dickeren und somit stabileren Blech gebildet sein.

Zur Kompensierung der thermischen Ausdehnung sind im oder am Halteboden, welcher den Zwischenraum zwischen den Thermoblechplattenmodulen und der überwiegend zylinderförmigen Hülle am unteren Ende der Thermoblechplattenmodule verschließt, vorteilhaft insbesondere ringförmige Kompensatoren vorgesehen. Besonders geeignet ist eine ringförmige Kompensation mit einem in Richtung senkrecht zur Ebene des Blechbodens betrachteten annähernd z-förmigen Profil. Gleichmaßen möglich sind jedoch auch herkömmliche, wellenförmige Kompensatoren.

Weiterhin sind bevorzugt auch in oder an der Blechabdeckung am oberen Ende des Zwischenraums zwischen Thermoblechplattenmodulen und überwiegend zylinderförmiger Hülle Kompensatoren für die axiale und/oder radiale Ausdehnung vorgesehen.

Jedes Thermoblechplattenmodul wird durch eine oder mehrere Verteileinrichtungen mit dem Wärmeträger versorgt. Dieser wird nach Durchströmen des Innenraums in den einzelnen Thermoblechplatten am anderen Ende des Thermoblechplattenmodules über eine oder mehrere Sammeleinrichtungen abgezogen. Da erfindungsgemäß ein Wärmeträger eingesetzt wird, der freiwerdende Reaktionswärme aufnimmt und hierbei zumindest teilweise verdampft, ist es zur Anpassung der Strömungsgeschwindigkeiten besonders vorteilhaft, pro Thermoblechplattenmodul jeweils eine Verteileinrichtung, jedoch zwei Sammeleinrichtungen vorzusehen.

Die Verteil- und Sammeleinrichtungen sind bevorzugt in der Weise ausgebildet, dass sie jeweils eine Kompensation für die Aufnahme der thermischen Ausdehnung der Thermoblechplattenmodule relativ zur umgebenden überwiegend zylinderförmigen Hülle aufweisen. Hier ist eine Kompensation beispielsweise durch eine geschwungene Rohrleitungsführung möglich.

Möglich ist es, zur Aufnahme der thermischen Ausdehnung der Thermoblechplattenmodule relativ zur umgebenden überwiegend zylinderförmigen Hülle eine geeignete bogenförmige oder Z- bzw. Omega-förmige geometrische Ausgestaltung der Verrohrung der Verteil- und Sammeleinrichtungen für den die Thermoblechplatten durchströmenden Wärmeträger zu gewährleisten. In einer weiteren Ausführungsform kann diese Kompensation durch axiale oder laterale Kompensatoren erfolgen, wobei eine gegebenenfalls erforderliche Rohrabstützung an einem inneren Tragwerk erfolgen kann.

Die Sammelrohre an den Thermoblechplatten für die Zufuhr und Verteilung sowie Sammlung und Abfuhr des Wärmeträgers werden besonders bevorzugt mit einer sogenannten Schlitzbodeneinschweißung wie folgt ausgeführt: Die einzelnen Thermoblechplatten eines Moduls werden zunächst mit einem zum Innenraum der Thermoblechplatten hin gewölbten rinnenförmigen Blech, das einen annähernd halbkreisförmigen Querschnitt sowie Öffnungen oder Schlitze für den Austritt des Wärmeträgers aufweist, verbunden. In diesem Zustand der Fertigung ist es möglich, die Schlitzbodeneinschweißungen sowohl in einer repräsentativen Stichprobe aber auch in vollem Umfang z.B. durch Röntgen auf Freiheit von Fertigungsfehlern zu prüfen. Anschließend wird dieses erste, annähernd rinnenförmige Blech, mit einem zweiten analog geformten Blech, jedoch mit entgegengesetzter Wölbung und ohne Öffnungen oder Schlitze, an den beiden Längsseiten verbunden, insbesondere durch Längsnahtschweißung, wobei ein rohrförmiges Bauteil mit annähernd kreisförmigem Querschnitt entsteht. Die beiden Enden dieses rohrförmigen Bauteiles werden durch Deckel verschlossen, die gegebenenfalls durch einen innenliegenden Zuganker verstärkt sein können.

In einer weiteren Ausführungsform ist auch die direkte Anschweißung von Rohrteilen mit eher kleiner Nennweite von z.B. 4 bis 30 mm an die Thermoblechplatten häufig an den Blechrändern zur Zu- bzw. Abfuhr des Wärmeträgers möglich.

Die Spalte zwischen den einzelnen Thermoblechplatten jedes Thermoblechplattenmodules dienen der Aufnahme des heterogenen partikelförmigen Katalysators.

Um ein Abfließen der Katalysatorpartikel aus den Spalten unter Einwirkung der Schwerkraft auszuschließen, müssen am unteren Ende derselben geeignete Katalysatorhalteroste vorgesehen sein. Dies kann z.B. mit Loch- oder Gitterblechen erfolgen, besonders vorteilhaft können hierfür so genannte Kantenspaltsiebe eingesetzt werden, die eine gute Rückhaltung des Katalysators bei gleichzeitig hoher Formstabilität und geringem Druckverlust für das durchströmende Reaktionsmedium gewährleisten.

Die Katalysatorhalteroste können beispielsweise schwenkbar eingebaut sein.

Besonders vorteilhaft ist es, wenn die Verteileinrichtungen für den Wärmeträger zu den Thermoblechplatten so eingebaut werden, dass die seitlichen Abstände von den Verteileinrichtungen zum Rand des Thermoblechplattenpaketes gleich sind, so dass nur ein einziger Typ von Katalysatorhalterost erforderlich ist. Vorteilhaft werden pro Thermoblechplattenmodul jeweils zwei Katalysatorhalterroste vorgesehen, das heißt beidseitig der Verteileinrichtung für den Wärmeträger.

Die Katalysatorhalteroste sind vorteilhaft so zu dimensionieren, dass sie über die Mannlöcher in der annähernd zylinderförmigen Hülle ein- und ausgebaut werden können. Häufig haben die Mannlöcher einen Innendurchmesser von 700 mm. Entsprechend ist eine Kantenlänge für die Katalysatorauflageroste von 650 mm bevorzugt.

In einer weiteren Ausführungsform ist es möglich diese Halteroste in kleinere Einheiten weiter zu unterteilen, aber auch jeden Spalt oder jede Spalthälfte, einzeln zu verschließen, so dass er auch getrennt entleert werden kann.

Alternativ ist es auch möglich, die Thermoblechplattenmodule vor dem Einbau derselben in den Reaktor, d.h. außerhalb des Reaktors, mit Katalysator zu befüllen.

Die die Thermoblechplattenmodule umgebende Hülle wurde vorstehend als überwiegend zylinderförmig bezeichnet. Darunter wird verstanden, dass sie einen Zylindermantel mit kreisförmigem Querschnitt aufweist, der an beiden Enden jeweils durch eine Haube verschlossen ist.

Die überwiegend zylinderförmige Hülle wird in der Regel vertikal aufgestellt.

Das fluide Reaktionsmedium wird über die eine Haube, häufig über die untere Haube, in den Reaktorinnenraum geleitet, durchströmt die mit dem heterogenen partikelförmigen Katalysator gefüllten Spalte zwischen den einzelnen Thermoblechplatten und wird am anderen Ende des Reaktors, über die andere, häufig die obere, Haube gezogen.

Die Hauben sind bevorzugt aus Edelstahl gefertigt oder mit Edelstahl-plattiert.

Die Hauben können fest verschweißt oder trennbar, beispielsweise über eine Flanschverbindung, mit dem Zylindermantel der Hülle verbunden sein. Die Flanschverbindung kann mittels eines Hydrauliksystems absenkbar ausgestaltet sein.

Die Hauben sind vorteilhaft über ein oder mehrere Mannlöcher, die in der Regel einen Durchmesser von 700 mm aufweisen, am Umfang begehbar. Hierzu ist ein erhöhter zylindrischer Schuss von Vorteil, der, wie auch die Haube, beispielsweise aus Edelstahl gefertigt oder mit Edelstahl-plattiert ist.

Über die Mannlöcher in den Hauben besteht Zugang zur Oberseite der Module, so dass der Katalysator einfach in die Spalte zwischen den Thermoblechplatten eingebracht werden kann, und zur Unterseite der Module, so dass die Halteroste leicht montiert und demontiert werden können.

Zum Ausbau des Katalysators können in der unteren Haube zusätzlich Vorrichtungen zum Haltern von Hilfsmitteln und zum Auffangen des Katalysators, die bereits beim Betrieb eingebaut sein können, sowie ein oder mehrere Stutzen zum Ablassen des Katalysators vorgesehen werden.

Als Werkstoff für den Zwischenraum zwischen den Thermoblechplattenmodulen und der Innenwand der überwiegend zylinderförmigen Hülle verschließenden Halteboden wie auch für die rechteckigen Stabilisierungskästen für die Thermoblechplattenmodule kann Kohlenstoffstahl verwendet werden. Alternativ ist es möglich, auch hierfür Edelstahl einzusetzen.

In einer oder beiden Hauben sind vorteilhaft Stutzen montiert, durch die Multithermoelemente in die einzelnen Thermoblechplattenmodule eingeführt werden können. Weiterhin können dort Stutzen für weitere Feldgeräte und Prozessmesseinrichtungen angebracht sein.

Bevorzugt sind im zylindrischen Mantel der überwiegend zylinderförmigen Hülle ein oder mehrere Kompensatoren zur Aufnahme vorzugsweise der axialen thermischen Ausdehnung vorgesehen.

Als Wärmeträgermedium kann Speisewasser eingesetzt werden, wie es typischerweise in Kraftwerken zur Dampferzeugung genutzt wird und dem Stand der Technik (Technische Regeln für Dampfkessel (TRD 611 vom 15. Oktober 1996 in BArbBl. 12/1996 s. 84, zuletzt geändert am 25. Juni 2001 in BArbBl. 8/2001 S. 108) entspricht. Typische Parameter des Speisewassers können sein: Leitfähigkeit kleiner 0,4 oder kleiner 0,2 Mikrosiemens/cm, Kalzium- und Magnesiumhärte kleiner 0,0005 Millimol je Liter oder unter der Nachweisgrenze, Natrium kleiner 5 Mikrogramm je Liter, Siliziumdioxid kleiner 20 Mikrogramm je Liter, Eisen kleiner 50 Mikrogramm je Liter und Sauerstoff kleiner 20 Mikrogramm je Liter und ein Gesamtgehalt an gelöstem Kohlenstoff von weniger als 0,2 Milligramm je Liter. Darüber hinaus sollte das Speisewasser arm oder frei an Halogen insbesondere Chlor sein. Es ist auch möglich, das Speisewasser z.B. durch Zugabe von Hilfsstoffen wie Hydrazin, Ammoniak gezielt zu konditionieren, insbesondere alkalisch zu stellen, weiterhin können dem Speisewasser Korrosionsinhibitoren zugesetzt werden.

Die obere Haube, durch die das Reaktionsmedium bei der oben beschriebenen bevorzugten Prozessführung den Reaktor verlässt, kann aus Kohlenstoffstahl bestehen.

Um den Zugang zu den Thermoblechplattenmodulen zwecks Reparatur oder Austausch zu gewährleisten, muss die obere Haube ebenfalls entfernt werden können. Wenn keine Flanschverbindung vorhanden ist, kann die obere Haube abgetrennt und nach Modulmontage wieder angeschweißt werden.

Eine Integrierung des aus den Thermoblechplatten abgezogenen Dampfes in unterschiedliche Dampfschienen ist möglich.

Der Reaktor kann optional an zwei Dampfschienen angeschlossen werden, davon eine mit höherem Druck, welche für die Aufheizung des Reaktors auf Betriebstemperatur genutzt werden kann.

Vorteilhaft ist der Betrieb an nur einer Dampfschiene.

Der Reaktor kann vorzugsweise mit Naturumlauf des Kühlmittels Wasser betrieben werden, wobei das Verhältnis von Speisewasser zu Dampf in der Regel 3 bis 12, vorzugsweise 5 bis 10, beträgt.

Möglich ist auch der Betrieb mit Zwangsumlauf, wobei dann eine breitere Lastvariation der Kühlung möglich ist. Das Speisewasser wird hierzu mit einem höheren Druck als im Kühlsystem vorhanden beispielsweise mittels einer Pumpe zugeführt.

Es kann eine Speisewasserumlaufgeschwindigkeit in den Verteileinrichtungen zwischen 0,5 und 3,0 m/s, oder auch von 1,0 bis 2,0 m/s und eine Wasserumlaufzahl zwischen 3 und 12 eingestellt werden. Die Strömungsgeschwindigkeit der Zweiphasenströmung (Dampf/Wasser) in den Sammeleinrichtungen kann zwischen 0,5 und 15 m/s, oder auch zwischen 2,0 und 6,0 m/s liegen.

Besonders bevorzugt führt man die Aufheizung der Thermoblechplattenmodule zur Inbetriebnahme des Reaktors aus demselben Wärmeträgernetz durch, in das beim Reaktionsbetrieb die Wärme durch zumindest teilverdampftes Wärmeträgermedium abgeführt wird.

Durch die Regelung des Dampfdruckes im Kühlsystem ist eine präzise Einstellung der Kühltemperatur möglich. Die Thermoblechplatten können erfahrungsgemäß bis zu einem Druck von etwa 80 bar im Kühlmittel betrieben werden. Der erfindungsgemäße Reaktor ermöglicht die Direktdampferzeugung bei Druckniveaus bis 80 bar.

Alternativ zur Ausführungsform mit Thermoblechplatten ist es jedoch auch möglich, eine, mehrere oder alle der gekühlten Reaktionszonen, insbesondere den Hauptreaktor und den Nachreaktor als einzelne Rohrbündelapparate oder auch als einzelne Zonen in einem einzigen Rohrbündelapparat auszubilden. Insbesondere kann die erste Reaktionszone als Rohrbündelapparat und die zweite oder die weiteren Reaktionszonen als Apparate mit Thermoblechplatten ausgeführt sein.

Bei der Ausführungsvariante mit Hauptreaktor und Nachreaktor als jeweils getrennte Rohrbündelapparate kann der Nachreaktor vorteilhaft mit größerem Rohrdurchmesser gegenüber dem Hauptreaktor ausgelegt sein. In allen Varianten mit Rohrbündelapparaten wird der Katalysator in der Regel in die Rohre als Schüttung eingebracht und die Reaktionswärme über einen Wärmeträger abgeführt, der durch den Zwischenraum um die Kontaktrohre zirkuliert.

Die erste Reaktionszone beziehungsweise der Hauptreaktor werden bevorzugt bei einer Temperatur des Kühlmittels beim Eintritt in die erste Reaktionszone bzw. in den Hauptreaktor im Bereich von 320 bis 380°C und die Temperatur des Kühlmittels beim Eintritt in die zweite oder die weiteren Reaktionszonen beziehungsweise in den Nachreaktor im Bereich von 250 bis 320°C, bevorzugt im Bereich von 270 bis 300°C, besonders bevorzugt im Bereich 280 bis 290°C, liegt.

In der zweiten oder den weiteren Reaktionszonen beziehungsweise im Nachreaktor werden 5 bis 10% des Gesamtumsatzes in der Anlage, bevorzugt bis 15%, besonders bevorzugt bis 20% des Gesamtumsatzes in der Anlage gefahren.

Nach dem erfindungsgemäßen Verfahren kann mit hohen Beladungen der Luft mit zu oxidierenden Edukten gefahren werden, insbesondere mit Beladungen von 30 bis 20 g o-Xylol je m³/h Reaktionsluft im Normzustand (0°C und 1,013 bar absolut), wobei typisch 60 bis 110 und sehr häufig 80 bis 105 g o-Xylol je m³/h Reaktionsluft im Normzustand zur Anwendung kommen.

Das erfindungsgemäße Verfahren ermöglicht eine hohe Flexibilität, indem auf unterschiedliche Lastbereiche ohne Ausbeuteverluste reagiert werden kann. Insbesondere können auch beispielsweise alterungsbedingte Performanceunterschiede von Hauptreaktorkatalysatoren ausgeglichen werden.

Bestehende Anlagen können zur Durchführung des erfindungsgemäßen Verfahrens in einfacher Weise nachgerüstet werden.

Es können einfach strukturierte Katalysatoren, insbesondere kostengünstige Standardkatalysatoren eingesetzt werden und es ist möglich, den Katalysator im Hauptreaktor auf Ausbeute zu optimieren. Insgesamt wird gegenüber bekannten Verfahren eine verbesserte Gesamtselektivität und -ausbeute erreicht.

Die Erfindung wird im Folgenden anhand einer Zeichnung näher erläutert:

Es zeigen im Einzelnen:
- Figur 1: schematische Darstellung einer ersten Ausführungsform einer erfindungsgemäßen Anlage mit Hauptreaktor und Nachreaktor,
- Figur 2: eine Variante der in Figur 1 dargestellten Ausführungsform,
- Figur 3: eine Ausführungsform mit Integrierung aller Reaktionszonen in einem einzigen Apparat,
- Figuren 4 bis 7: weitere Varianten der in Figur 3 dargestellten Ausführungsform,
- Figur 8: eine weitere Variante der in Figur 1 dargestellten Ausführungsform,
- Figur 9A und 9B: die schematische Darstellung bevorzugter Ausführungsformen mit Hauptreaktor und Nachreaktor mit Schnittdarstellung in Figur 9C und
- Figuren 10 bis 13: unterschiedliche Ausführungsformen für die Anordnung von Thermoblechplat- ten.

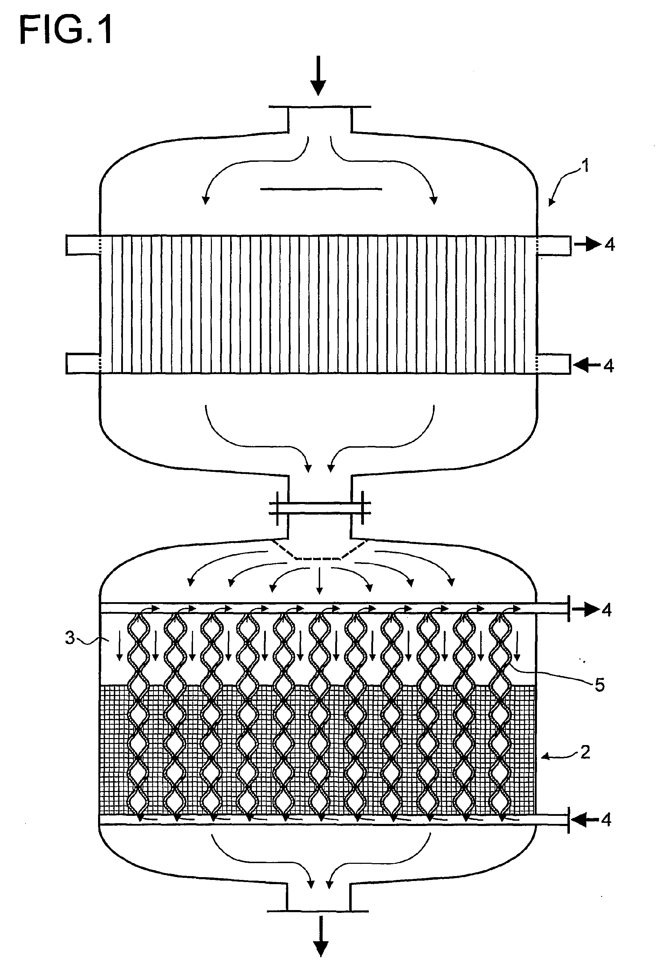

Die Ausführungsform in Figur 1 zeigt die schematische Darstellung einer Anlage mit einem Hauptreaktor 1 und einem daran angeflanschten Nachreaktor 2. Der Hauptreaktor 1 ist als Rohrbündelapparat ausgebildet, durch dessen Kontaktrohre das Reaktionsgasgemisch, durch Pfeile angedeutet, von oben nach unten strömt. Durch den Zwischenraum zwischen den Kontaktrohren strömt das Kühlmittel 4.

Der Nachreaktor 2 ist als Apparat mit Thermoblechplatten 5 ausgebildet durch die ebenfalls ein Kühlmittel 4 strömt. Im Zwischenraum zwischen den Thermoblechplatten 5 ist im unteren Bereich ein Feststofflcatalysator eingefüllt. Aus der Darstellung in Figur 1 ist zu erkennen, dass im oberen Bereich des Nachreaktors 2 der Raum zwischen den Thermoblechplatten 5 frei ist. Dadurch fungiert dieser Teil des Nachreaktors 2 als integrierte Vorrichtung 3 zur Zwischenkühlung.

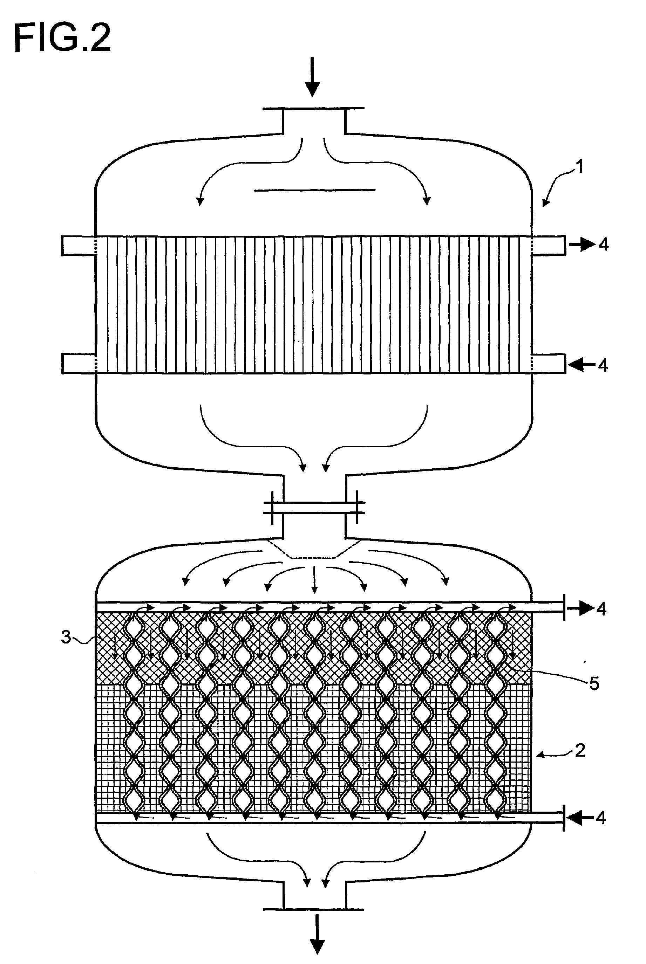

Die Ausführungsform in Figur 2 unterscheidet sich von der in Figur 1 dargestellten Ausführungsform dadurch, dass in den als Zwischenkühler 3 fungierenden Bereich des Nachreaktors 2 ein Inertmaterial eingefüllt ist.

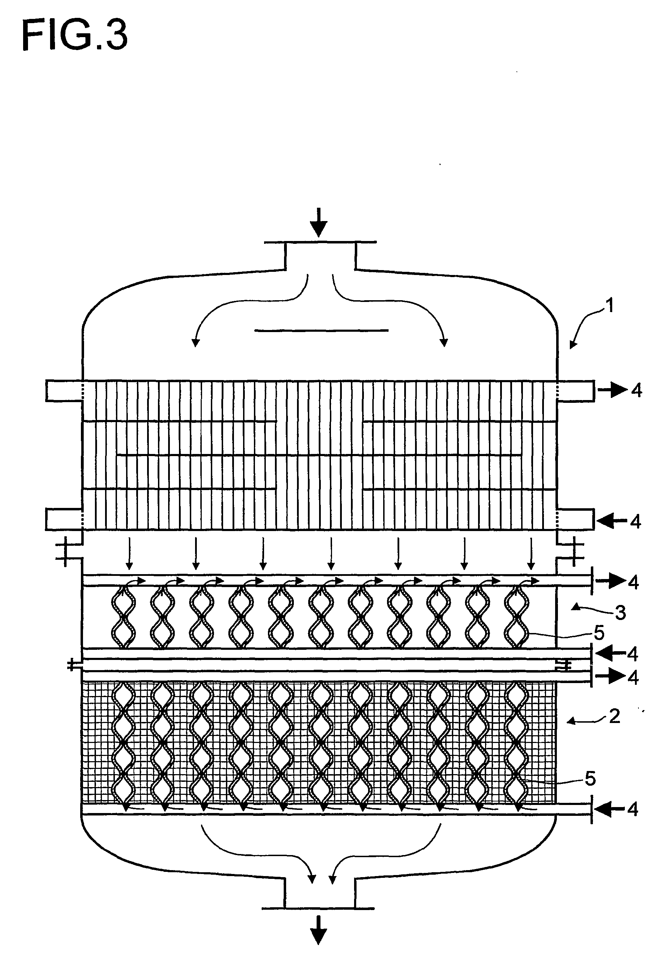

Figur 3 zeigt eine Ausführungsform mit in einem einzigen Apparat integrierten Hauptreaktor 1, Nachreaktor 2 und Vorrichtung zur Zwischenkühlung 3, wobei die Vorrichtung zur Zwischenkühlung 3 und der Nachreaktor 2 jeweils mit Thermoblechplatten 5 ausgestattet sind. Hauptreaktor 1, Nachreaktor 2 und Vorrichtung zur Zwischenkühlung 3 haben jeweils getrennte Kreisläufe für das Kühlmittel 4. Der Hauptreaktor 1 ist mit einem Bündel von Kontaktrohren sowie mit Umlenkblechen ausgestattet.

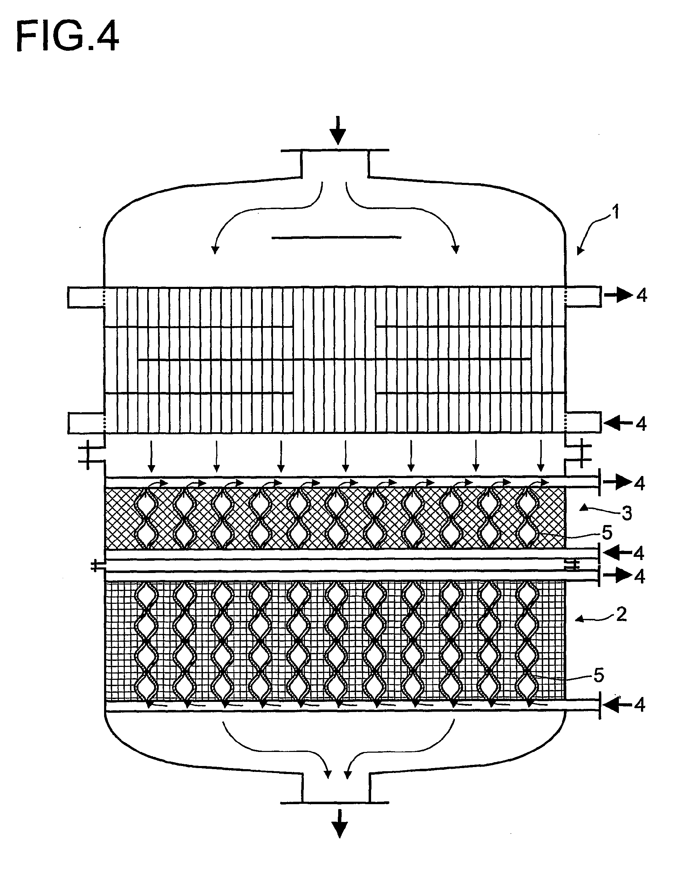

Figur 4 unterscheidet sich von der in Figur 3 dargestellten Ausführungsform nur dadurch, dass in der Vorrichtung zur Zwischenkühlung 3 zwischen den Thermoblechplatten 5 Inertmaterial eingebracht ist.

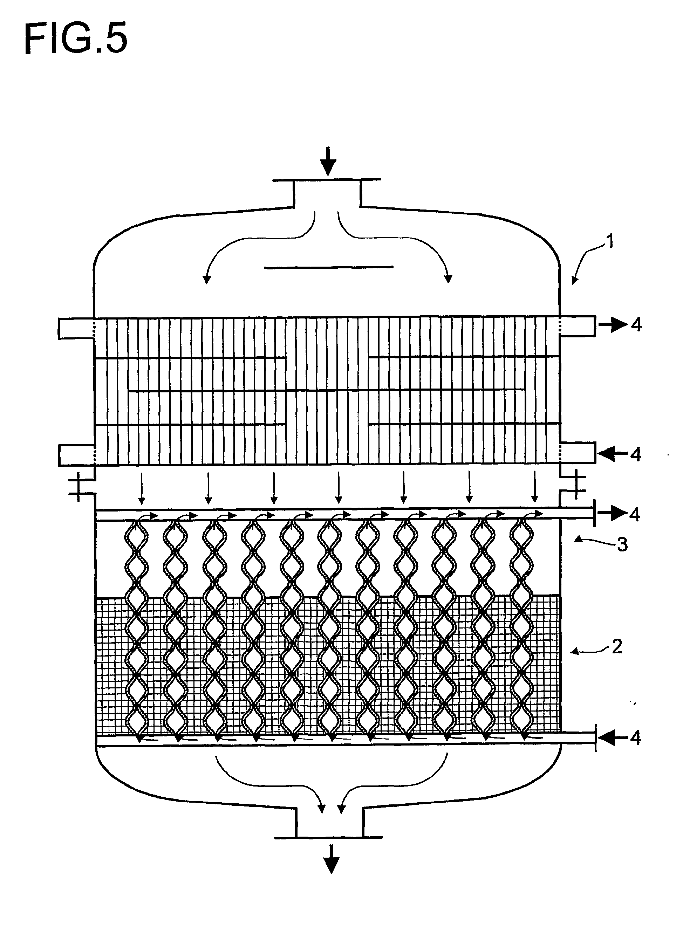

Die Ausführungsform in Figur 5 unterscheidet sich von der in Figur 3 dargestellten Ausführungsform dadurch, dass die Vorrichtung zur Zwischenkühlung 3 in den Nachreaktor 2 integriert ist.

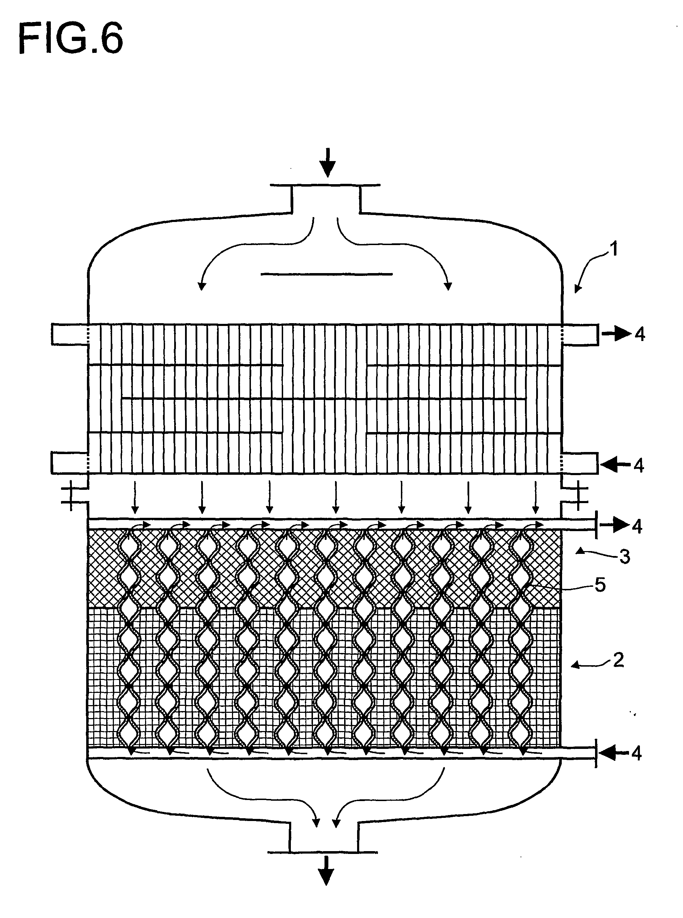

Die in Figur 6 dargestellte Ausführungsform unterscheidet sich von der Ausführungsform in Figur 5 nur dadurch, dass in dem Bereich, der als Vorrichtung zur Zwischenkühlung fungiert zwischen den Thermoblechplatten 5 ein Inertmaterial eingebracht ist.

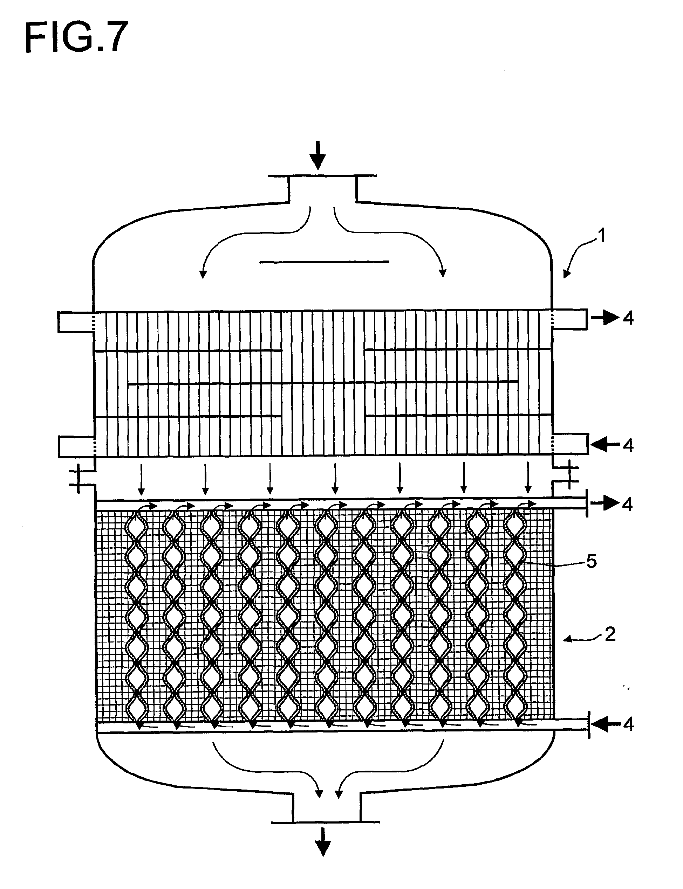

Die Ausführungsform in Figur 7 unterscheidet sich von der Ausführungsform in Figur 6 dadurch, dass der Nachreaktor 2 der Bereich zwischen den Thermoblechplatten 5 vollständig mit Feststoffkatalysator gefüllt ist.

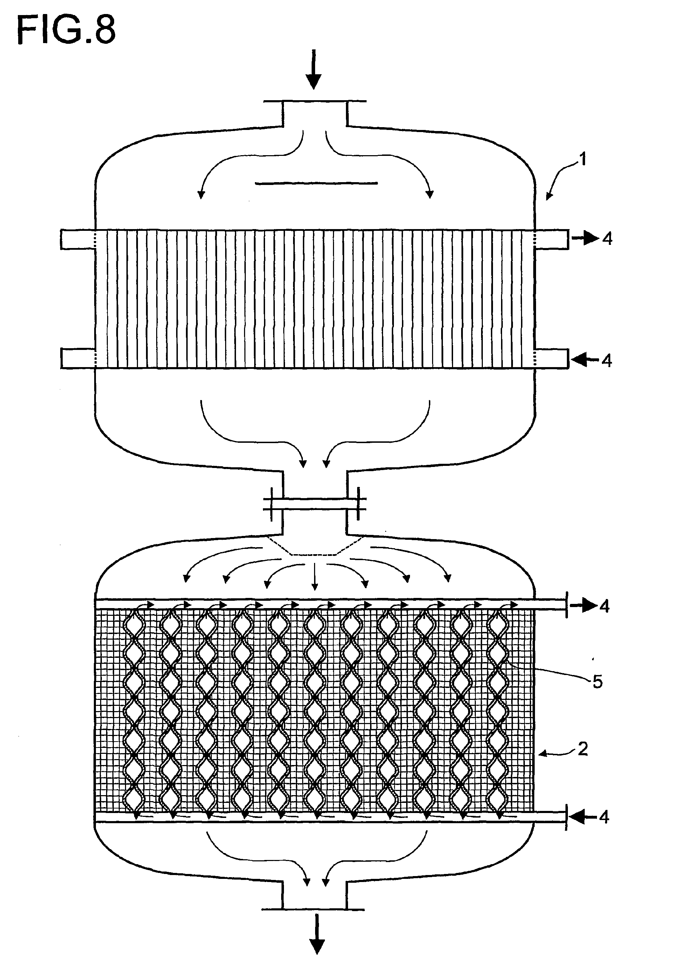

Auch in Figur 8 ist der Bereich zwischen den Thermoblechplatten 5 im Nachreaktor 2 vollständig mit einem festen Katalysatormaterial ausgefüllt.

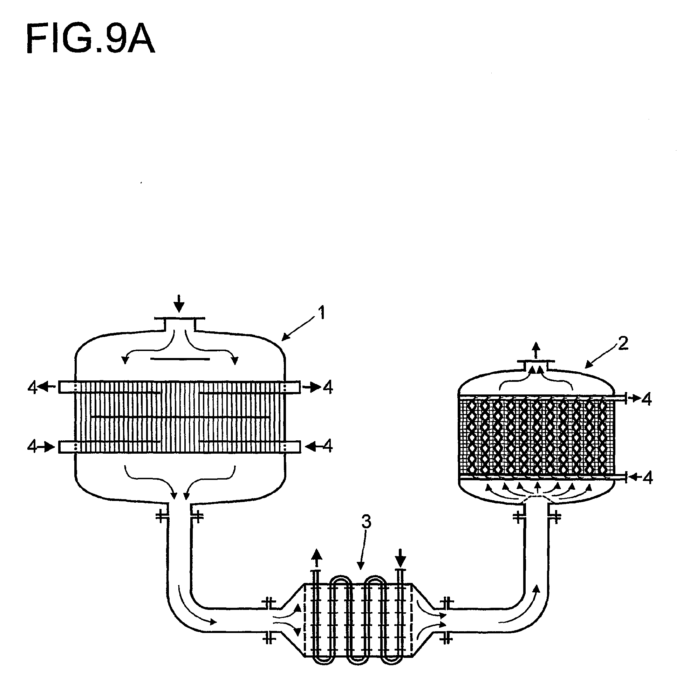

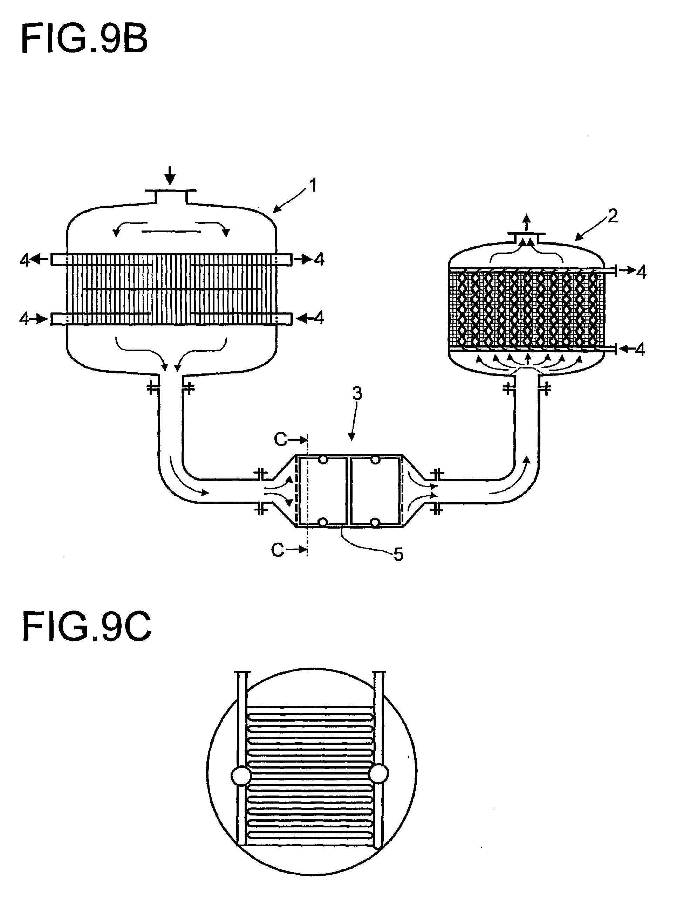

Die Ausführungsformen in Figur 9A und 9B zeigen eine Anlage mit nebeneinander angeordnetem Hauptreaktor 1 und Nachreaktor 2, wobei im Zwischenkühler 3 eine Kühlschlange (Fig. 9A) bzw. Thermoblechplatten (Fig 9B) angeordnet sind. Figur 9C zeigt den Schnitt C-C durch den Zwischenkühler 3 aus Figur 9B.

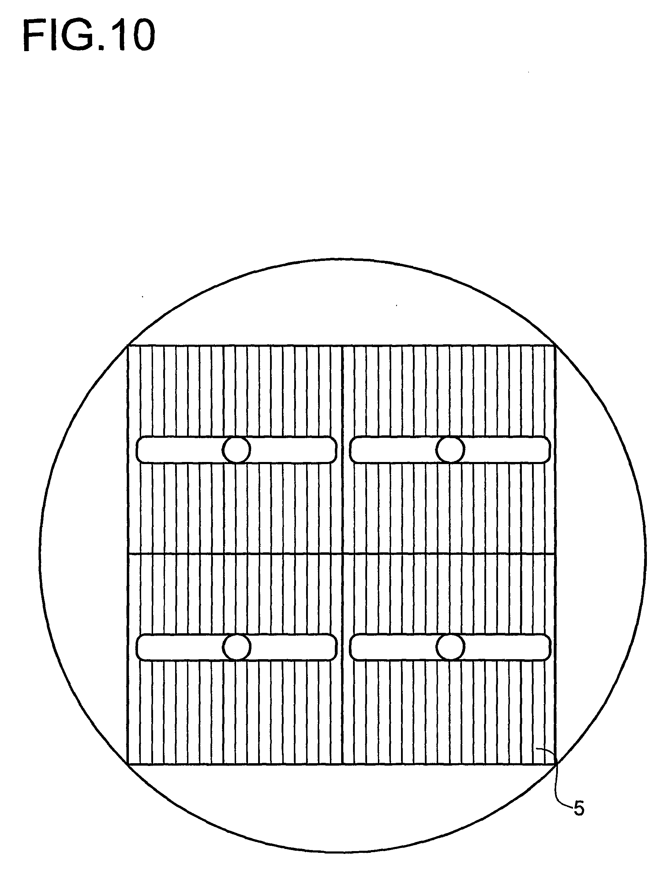

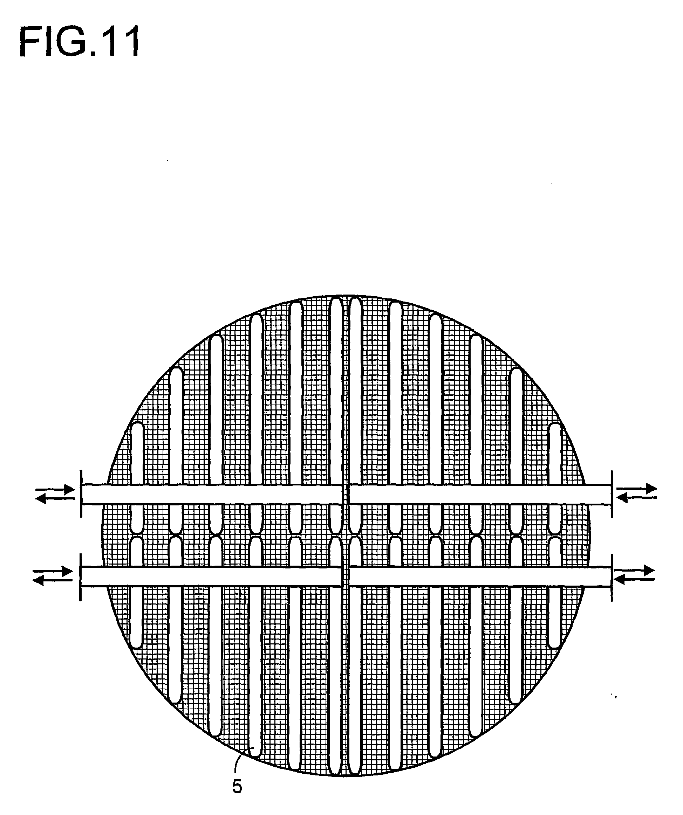

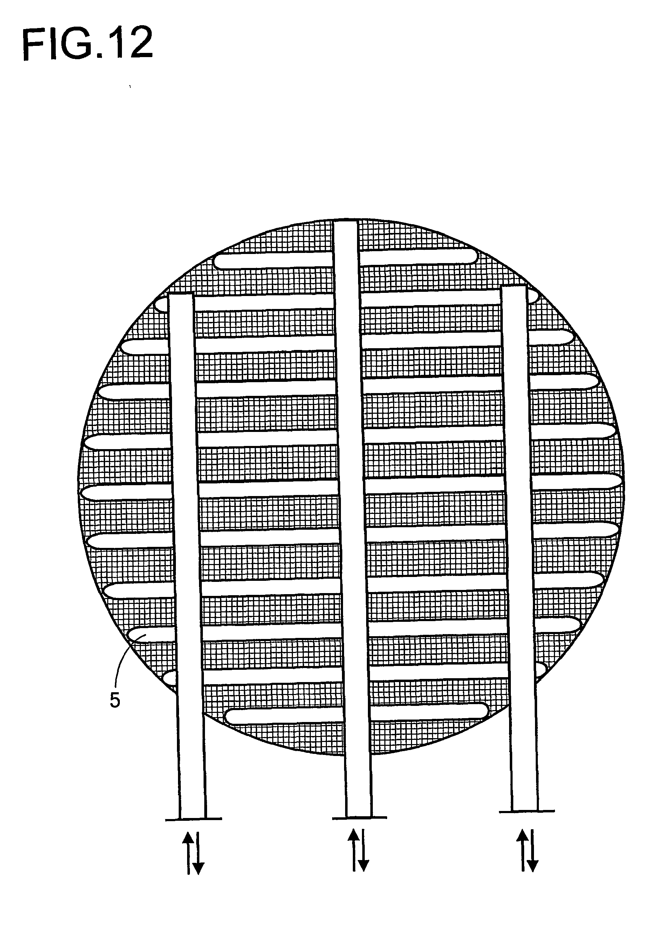

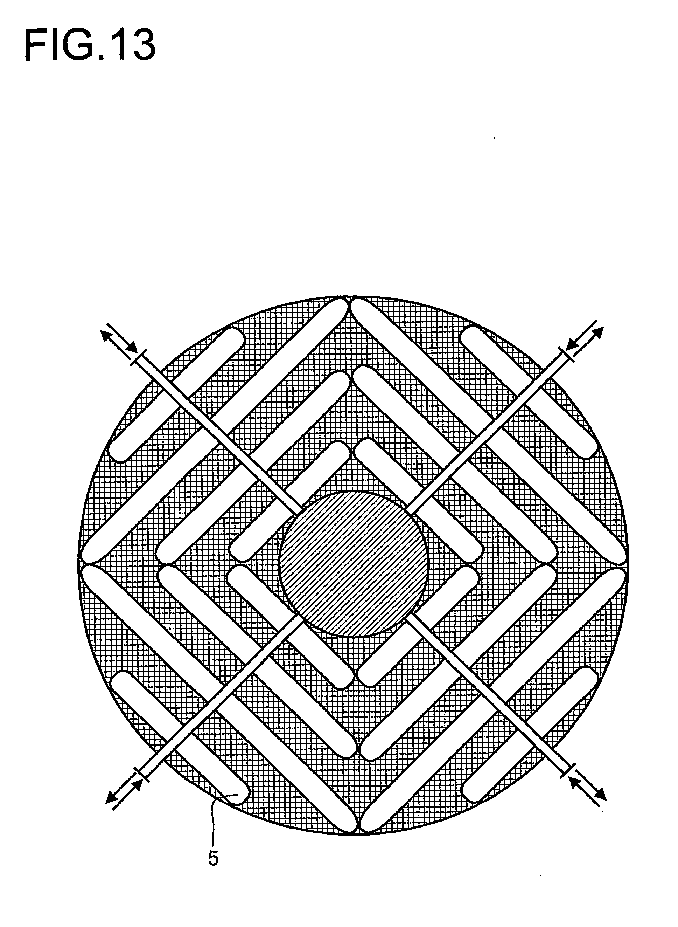

Die Figuren 10 bis 13 zeigen verschiedene Anordnungen von Thermoblechplatten 5 in der zur Durchführung des erfindungsgemäßen Verfahrens eingesetzten Anlage.

## Patentansprüche

1. Verfahren zur Herstellung von Phthalsäureanhydrid durch katalytische Partialoxidation von o-Xylol und/oder Naphthalin mit einem Sauerstoff enthaltenden Gas in einer Anlage mit
- zwei oder mehreren mit einem Kühlmittel gekühlten Reaktionszonen,
- einer oder mehreren, zwischen den Reaktionszonen angeordneten Vorrichtungen zur Zwischenkühlung des Reaktionsgemisches zwischen den Reaktionszonen, wobei die Temperatur des Kühlmittels beim Eintritt in die zweite oder in die weiteren Reaktionszonen gegenüber der Temperatur des Kühlmittels beim Eintritt in die erste Reaktionszone abnimmt,
**dadurch gekennzeichnet, dass** die Temperatur des Kühlmittels beim Eintritt in die erste Reaktionszone um mehr als 20° höher ist als die Temperatur des Kühlmittels beim Eintritt in die zweite oder in die weiteren Reaktionszonen.

2. Verfahren nach Anspruch 1, **dadurch gekennzeichnet, dass** die Anlage zwei gekühlte Reaktionszonen aufweist, und zwar einen gekühlten Hauptreaktor und einen gekühlten Nachreaktor.

3. Verfahren nach Anspruch 2, **dadurch gekennzeichnet, dass** die Temperatur des Kühlmittels beim Eintritt in den Hauptreaktor im Bereich von 320 bis 380°C und die Temperatur des Kühlmittels beim Eintritt in den Nachreaktor im Bereich von 250 bis 320°C, liegt.

4. Verfahren nach Anspruch 2 oder 3, **dadurch gekennzeichnet, dass** im Nachreaktor ein Restumsatz von 5 bis 10%, bezogen auf den Gesamtumsatz in der Anlage gafahren wird.

5. Verfahren nach einem der Ansprüche 1 bis 4, **dadurch gekennzeichnet, dass** die Wärmeabführung durch Siedekühlung des Kühlmittels erfolgt.

6. Verfahren nach einem der Ansprüche 1 bis 5, **dadurch gekennzeichnet, dass** die zwei oder mehreren gekühlten Reaktionszonen sowie die zwischen den Reaktionszonen angeordneten Vorrichtung(en) zur Zwischenkühlung des Reaktionsgasgemisches in einem einzigen Apparat mit Thermoblechplatten, die von einem Kühlmittel durchströmt sind, zusammengefasst sind.

7. Verfahren nach Anspruch 6, **dadurch gekennzeichnet, dass** der Apparat einen Hauptreaktor, einen Zwischenkühler und einen Nachreaktor umfasst und dass ein erster Kühlkreis für den Hauptreaktor und ein zweiter, gemeinsamer Kühlkreis, für den Zwischenkühler und den Nachreaktor vorgesehen ist.

8. Verfahren nach einem der Ansprüche 1 bis 7, **dadurch gekennzeichnet, dass** in der oder den Vorrichtungen zur Zwischenkühlung eine Inertschüttung vorgesehen ist.

9. Verfahren nach einem der Ansprüche 1 bis 5, **dadurch gekennzeichnet, dass** eine, mehrere oder alle der gekühlten Reaktionszonen jeweils als Rohrbündelapparate ausgeführt sind.

10. Verfahren nach Anspruch 9, **dadurch gekennzeichnet, dass** die erste Reaktionszone als Rohrbündelapparat und die zweite oder die weiteren Reaktionszonen als Apparate mit Thermoblechplatten ausgeführt sind.

## Claims

1. A process for preparing phthalic anhydride by catalytic partial oxidation of o-xylene and/or naphthalene with a gas comprising oxygen in a plant comprising
- two or more reaction zones cooled with a coolant,
- one or more devices arranged between the reaction zones for intermediate cooling of the reaction mixture between the reaction zones, the temperature of the coolant on entry into the second or into the further reaction zones decreasing compared to the temperature of the coolant on entry into the first reaction zone,
wherein the temperature of the coolant on entry into the first reaction zone is more than 20° higher than the temperature of the coolant on entry into the second or into the further reaction zones.

2. The process according to claim 1, wherein the plant has two cooled reaction zones, specifically a cooled main reactor and a cooled postreactor.

3. The process according to claim 2, wherein the temperature of the coolant on entry into the main reactor is in the range from 320 to 380°C and the temperature of the coolant on entry into the postreactor is in the range from 250 to 320°C.

4. The process according to claim 2 or 3, wherein the postreactor is operated at a residual conversion of from 5 to 10% based on the overall conversion in the plant.

5. The process according to any of claims 1 to 4, wherein the heat is removed by evaporative cooling of the coolant.

6. The process according to any of claims 1 to 5, wherein the two or more cooled reaction zones and also the device(s) arranged between the reaction zones for intermediate cooling of the reaction gas mixture are combined in a single apparatus with thermoplates which are flowed through by a coolant.

7. The process according to claim 6, wherein the apparatus comprises a main reactor, an intermediate cooler and a postreactor, and a first cooling circuit for the main reactor and a second, combined cooling circuit for the intermediate cooler and the postreactor are provided.

8. The process according to any of claims 1 to 7, wherein an inert bed is provided in the apparatus(es) for intermediate cooling.

9. The process according to any of claims 1 to 5, wherein one, more or all of the cooled reaction zones are each designed as tube bundle apparatuses.

10. The process according to claim 9, wherein the first reaction zone is designed as a tube bundle reactor and the second reaction zone or the further reaction zones as apparatuses with thermoplates.

## Revendications

1. Procédé de fabrication d'anhydride de l'acide phtalique par oxydation partielle catalytique d'o-xylène et/ou de naphtaline avec un gaz contenant de l'oxygène dans une installation comprenant
- deux zones de réaction ou plus, refroidies avec un agent de refroidissement,
- un ou plusieurs dispositifs placés entre les zones de réaction pour le refroidissement intermédiaire du mélange réactionnel entre les zones de réaction, la température de l'agent de refroidissement déclinant lors de l'entrée dans la seconde zone de réaction ou les zones de réaction ultérieures par rapport à la température de l'agent de refroidissement lors de l'entrée dans la première zone de réaction,
**caractérisé en ce que** la température de l'agent de refroidissement lors de l'entrée dans la première zone de réaction est supérieure de plus 20 °C à la température de l'agent de refroidissement lors de l'entrée dans la seconde zone de réaction ou les zones de réaction ultérieures.

2. Procédé selon la revendication 1, **caractérisé en ce que** l'installation comporte deux zones de réaction refroidies, à savoir un réacteur principal refroidi et un réacteur secondaire refroidi.

3. Procédé selon la revendication 2, **caractérisé en ce que** la température de l'agent de refroidissement lors de l'entrée dans le réacteur principal se situe dans la plage allant de 320 à 380 °C et la température de l'agent de refroidissement lors de l'entrée dans le réacteur secondaire dans la plage allant de 250 à 320 °C.

4. Procédé selon la revendication 2 ou 3, **caractérisé en ce qu'**une conversion résiduelle de 5 à 10 %, par rapport à la conversion totale dans l'installation, est réalisée dans le réacteur secondaire.

5. Procédé selon l'une quelconque des revendications 1 à 4, **caractérisé en ce que** la dissipation de chaleur à lieu par refroidissement par ébullition de l'agent de refroidissement.

6. Procédé selon l'une quelconque des revendications 1 à 5, **caractérisé en ce que** les deux zones de réaction refroidies ou plus, ainsi que le ou les dispositifs placés entre les zones de réaction pour le refroidissement intermédiaire du mélange gazeux réactionnel, sont groupés dans un appareil unique muni de thermoplaques qui sont traversées par un agent de refroidissement.

7. Procédé selon la revendication 6, **caractérisé en ce que** l'appareil comporte un réacteur principal, un refroidisseur intermédiaire et un réacteur secondaire, et **en ce qu'**un premier circuit de refroidissement est prévu pour le réacteur principal et un second circuit de refroidissement commun pour le refroidisseur intermédiaire et le réacteur secondaire.

8. Procédé selon l'une quelconque des revendications 1 à 7, **caractérisé en ce qu'**un garnissage inerte est prévu dans le ou les dispositifs pour le refroidissement intermédiaire.

9. Procédé selon l'une quelconque des revendications 1 à 5, **caractérisé en ce qu'**une, plusieurs ou toutes les zones de réaction refroidies sont chacune conçues sous la forme d'appareils à faisceau de tubes.

10. Procédé selon la revendication 9, **caractérisé en ce que** la première zone de réaction est conçue sous la forme d'un appareil à faisceau de tubes et la seconde zone de réaction ou les zones de réaction ultérieures sont conçues sous la forme d'appareils munis de thermoplaques.
